# EUROPÄISCHE PATENTSCHRIFT

(11) **EP 3 895 809 B1**
(45) Veröffentlichungstag und Bekanntmachung des Hinweises auf die Patenterteilung: **06.03.2024**
(21) Anmeldenummer: 20169402.3
(22) Anmeldetag: 14.04.2020
(51) Int. Cl.: B05B 11/00, G16H 40/63, B05B 12/00, G16H 40/00, G16H 20/10, G16H 40/67, B05B 11/10

(54) **VERFAHREN ZUR AUSWERTUNG EINER PUMPBETÄTIGUNG SOWIE FLÜSSIGKEITSSPENDER UND AUSWERTUNGSEINHEIT, DIE DIESES VERFAHREN NUTZEN**
METHOD FOR EVALUATING A PUMP ACTUATION AND LIQUID DISPENSER AND EVALUATION UNIT USING THIS METHOD
PROCÉDÉ D'ÉVALUATION D'UN MOUVEMENT DE POMPAGE AINSI QUE DISTRIBUTEUR DE LIQUIDE ET UNITÉ D'ÉVALUATION UTILISANT LEDIT PROCÉDÉ

(43) Veröffentlichungstag der Anmeldung: 20.10.2021
(73) Patentinhaber: Aptar Radolfzell GmbH, 78315 Radolfzell (DE)
(72) Erfinder: KARGE, Marius, 78333 Stockach (DE); SCHMID, Kevin, 78462 Konstanz (DE)
(74) Vertreter: Witte, Weller und Partner Patentanwälte mbB Stuttgart

(56) Entgegenhaltungen:
- EP-A1- 3 636 348
- WO-A1-2014/106096
- DE-B3-102011 079 950
- DE-B3-102014 204 939
- FR-A1- 2 861 460

## Beschreibung

Die Erfindung betrifft den Bereich der Pumpspender zum Austrag von Flüssigkeit, insbesondere der Pumpspender im medizinischen Bereich, die mit einer pharmazeutischen Flüssigkeit befüllt sind. Bei solchen Pumpspendern ist vorgesehen, dass durch manuelle Betätigung einer Pumpe des Pumpspenders Flüssigkeit aus einem Flüssigkeitsspeicher durch eine Austragöffnung ausgetragen werden kann, beispielsweise in Form eines zerstäubten Sprühstrahls, eines unzerstäubten Jets oder in Form von Einzeltropfen.

Pumpspenderwerden von Benutzern verwendet, um pharmazeutische Flüssigkeiten zu applizieren, beispielsweise auf nasalem oder oralem Wege und insbesondere auch sublingual, also unterhalb der Zunge. Insbesondere bei unangenehm schmeckenden oder riechenden pharmazeutischen Flüssigkeiten besteht die Gefahr, dass der Benutzer keinen vollständigen Austrag durchführt, sondern stattdessen die Betätigung zu früh zu beenden. Folge ist eine ungenügende Menge an ausgetragener pharmazeutischer Flüssigkeit.

Aus Dokument EP 3636348 A1 sind verschiedene Gestaltungen von Auswerteeinheiten für Pumpspender bekannt, die dem Zweck dienen, eine Betätigung des Pumpspenders zu erfassen. Es finden hierfür beispielsweise Weg- oder Kraft- bzw. Drucksensoren Verwendung, insbesondere ein Taster.

Aus Dokument DE 102014204939 B3 ist es ebenfalls bekannt, die Betätigung eines Spenders zu erfassen und aus einer erfassten Kraft oder einem erfassten Druck oder dem Schalten eines Tasters auf die Betätigung zu schließen.

Aus Dokument WO 2014/106096 A1 ist ein Adapter zur Anbringung an einem Spender bekannt, der einen Sensor zur Krafterfassung umfassen kann. Wird bei einer Betätigung zu wenig Kraft verwendet, so kann dies in einem Speicher des Adapters gespeichert werden.

### AUFGABE UND LÖSUNG

Aufgabe der Erfindung ist es, ein Verfahren und Vorrichtungen zur Durchführung des Verfahrens zur Verfügung zu stellen, die die Gefahr unvollständigen Austrags verringert oder verhindert.

Erfindungsgemäß wird hierzu ein Verfahren zur Auswertung einer Pumpbetätigung an einem Pumpspender zum Austrag einer Flüssigkeit, insbesondere einer pharmazeutischen Flüssigkeit, vorgeschlagen, welches Anwendung findet bei Nutzung eines Pumpspenders, der über zwei gegeneinander manuell bewegbare Teileinheiten verfügt und der über eine Pumpe mit einer durch die Bewegung der Teileinheiten von einer unbetätigten ersten Endlage in eine betätigte zweite Endlage verkleinerbare Pumpkammer verfügt. Die vorzugsweise pharmazeutische Flüssigkeit gelangt aus dem vorzugsweise zwischen 20 ml und 1000 ml großen Flüssigkeitsspeicherzur Pumpe und von dort weiter zu einer Austragöffnung. Die im Flüssigkeitsspeicher gelagerter Flüssigkeit kann insbesondere ein Pharmazeutikum, insbesondere mit pharmazeutisch aktiven Substanzen, aber insbesondere Allergenextrakten sein.

Die genannten beiden Teileinheiten, bei denen es sich insbesondere um eine Basis mit Flüssigkeitsspeicher einerseits und eine Betätigungseinheit mit Betätigungsdrücker und Austragöffnung andererseits handeln kann, werden im Zuge des Austrags aufeinander zu gedrückt, wobei durch das Zusammendrücken der Teileinheiten die Pumpe betätigt wird. Bei vielen Pumpspendern ist vorgesehen, dass diese einhändig verwendet werden, indem ihr Flüssigkeitsspeicher mit der Hand umgriffen wird und ein Betätigungsdrücker mit einem Finger der Hand, üblicherweise dem Zeigefinger, niedergedrückt wird.

Die Austragöffnung kann insbesondere seitlich an der Betätigungseinheit vorgesehen sein, so dass eine Austragrichtung mit der Betätigungsrichtung der Betätigungseinheit einen Winkel > 0°, vorzugweise zwischen 60° und 120° einschließt. Zur besseren Heranführung der Austragöffnung an die Applikationsstelle kann eine Gestaltungvon Vorteil sein, bei der der ein frei hervorragendes Applikatorrohr vorgesehen ist, an dessen distalem Ende die Austragöffnung angeordnet ist.

Die Betätigungseinheit kann insbesondere auch zursublingualen Applikator vorgesehen sein. In diesem Falle ist vorzugsweise eine angewinkelte Applikatorrohrspitze am Ende des seitlich von der Betätigungseinheit hervorstehenden Applikatorrohres vorgesehen.

Die Pumpe kann insbesondere eine Kolbenpumpe sein, die als Kolbenpumpe mit einem Pumpenzylinder und einem darin verschieblichen Pumpenkolben ausgebildet ist. Der Pumpenzylinder und der Pumpenkolben begrenzen gemeinsam die Pumpenkammer, wobei vorzugsweise eingangsseitig und ausgangseitig der Pumpenkammer druckabhängig öffnende und schließende Ventile vorgesehen sind. Wenn die Teileinheiten aufeinander zu verschoben wird, schließt das Einlassventil, das Ausgangsventil öffnet und die mit der Bewegung der Teileinheiten verbundene Bewegung des Pumpenkolbens im Pumpenzylinder verdrängt Flüssigkeit, die durch das Auslassventil zur Austragöffnung gefördert und hier ausgetragen wird. Beim Rückhub schließt das Auslassventil, das Einlassventil öffnet und Flüssigkeit wird aus dem Flüssigkeitsspeicher in die Pumpkammer gesogen.

Neben Kolbenpumpen können jedoch auch anderweitige Pumpen eingesetzt werden, die zum Zwecke des Pumpens in eine Richtung bis in eine Endlage kraftbeaufschlagbar sind und die beim Rückhub in ihre Ausgangsposition zurückkehren. Hierzu gehören beispielsweise auch Balgpumpen.

Zur Erfassung der betätigten zweiten Endlage wird erfindungsgemäß während der Bewegung der Teileinheiten zueinander mittels eines Kraftsensors die Kraft erfasst, mit der die Teileinheiten manuell aufeinander zu bewegt werden. Diese Kraft ist jene Kraft, die der Benutzer aufbringt, insbesondere zwischen seiner die eine Teileinheit umgreifenden Hand und dem die anderen Teileinheit demgegenüber niederdrückenden Finger, insbesondere dem Zeigefinger.

Ein Kraftsensor im Sinne der Erfindung ist ein Sensor, der die anliegende Kraft in Form eines analogen Kraftwertes zurückgibt, also eines Wertes, der umso höher ist, je höher die vom Benutzer ausgeübte Kraft ist. Ein einfacher Schalter ist dementsprechend kein Kraftsensor im Sinne der Erfindung. Allerdings sind im Sinne der Erfindung auch solche Sensoren Kraftsensoren, die einen unmittelbar auf die anliegende Kraft rückführbaren Messwert zurückgeben, so insbesondere auch ein Drucksensor oder ein mit einer rückstellenden Federkraft gekoppelter Wegsensor.

Der Kraftsensor kann insbesondere vorzugsweise FSR-Sensor (Force Sensitive Resistor) oder als Piezo-Sensor ausgebildet sein. Diese liefern analoge Sensorwerte in Form variabler Widerstände oder Spannungen.

Dem Kraftsensor ist vorzugsweise eine rückstellende Federkraft zugeordnet, die bei Wegfall der Betätigungskraft die auf den Kraftsensor wirkenden Flächen der Auswertungseinheit in eine Ausgangslage zurückdrücken. Insbesondere vorzugsweise ist der Kraftsensor in der Ausgangslage bedingt durch die Federkraft von einer der auf ihn wirkenden Flächen entkoppelt, beispielsweise durch einen schmalen Spalt, so dass der Kraftsensor in dieser Ausgangslage zuverlässig ein Null-Signal liefert.

Die vom Kraftsensor gemeldeten Kraftsensorwerte werden erfindungsgemäß dahingehend ausgewertet, dass ein für das Erreichen der betätigten zweiten Endlage charakteristischer Kraftanstieg erkannt wird. Wie dies erfolgen kann, wird im Weiteren noch erläutert. Ergebnis der Auswertung ist, dass erkannt wird, ob der fragliche charakteristische Kraftanstieg zu verzeichnen war, der sich daraus ergibt, dass die Pumpe ihre Endlage erreicht hat, insbesondere dass der Pumpenkolben bis in seine tiefste Endlage in den Pumpenzylinder eingefahren ist. Es hat sich gezeigt, dass das Erreichen dieser Endlage durch den Benutzer unbeabsichtigt dazu führt, dass mit einer stärkeren und erkennbaren Betätigungskraft betätigt wird.

In Abhängigkeit davon, ob der für das Erreichen der betätigten zweiten Endlage charakteristischer Kraftanstieg erfasst wurde, wird vorzugsweise anschließend ein für das Ergebnis charakteristisches Signal durch eine Ausgabeeinrichtung haptisch, akustisch und/oder visuell ausgegeben. Die Ausgabe erfolgt dabei vorzugsweise am Pumpenspender selbst oder an einer an diesem befestigten Auswertungseinheit. Ein haptisches Signal ist beispielsweise ein Vibrationssignal. Ein visuelles Signal kann insbesondere durch ein Display oder eine LED erzeugt werden, wobei gegebenenfalls unterschiedliche Signale mit unterschiedlichen Farben oder Blinksequenzen unterschieden werden können. Ein akustisches Signal kann insbesondere in einem oder mehreren unterschiedlichen Signaltönen bestehen.

Im einfachsten Falle wird ein Signal als positive Rückmeldung ausgegeben, wenn der für das Erreichen der zweiten betätigten Hubendlage charakteristischer Verlauf der Kraft erkannt wurde. Es könnte sich beispielsweise um ein grünes Aufleuchten einer LED oder eine Vibration handeln. Alternativ oder zusätzlich kann vorgesehen sein, dass ein Signal als negative Rückmeldung ausgegeben wird, wenn nach Erfassung des Beginn des Austrags kein für die zweite betätigte Hubendlage charakteristischer Verlauf der Kraft erkannt wird, wobei Auslöser dieses Signals der Ablauf einer vorgegebenen Zeitspanne ab Beginn der Betätigung sein kann. Alternativ kann ein Abfallen des Kraftsensorwertes als Wegfall der Betätigungskraft erkannt werden. Eine LED könnte das negative Ergebnis beispielsweise durch rotes Aufleuchten der LED anzeigen.

Weiterhin ist es auch möglich, bereits während der Betätigung ein Signal auszugeben, um dem Benutzer zu signalisieren, dass der Beginn der Betätigung erkannt wurde, insbesondere wenn der Kraftsensorwert ein vorgegebenes Minimum überschritten hat. Ein solches Signal endet, sobald der für das Erreichen der betätigten zweiten Endlage charakteristischer Kraftanstieg erkannt wird und/oder sobald der Abbruch der Betätigung erkannt wird, und entsprechend eine positive oder eine negative Rückmeldung als Signal ausgegeben wird.

Die Signalisierung, insbesondere die unmittelbare Signalisierung am Pumpspender oder an einer daran befestigten Auswertungseinheit, wird als bevorzugte Nutzung des Ergebnisses der Auswertungangesehen. Es kann jedoch stattdessen oder vorzugsweise zusätzlich vorgesehen sein, dass das Ergebnis der Auswertung über eine drahtlose Schnittstelle an ein externes Darstellungsgerät gesendetwird. Bei der drahtlosen Schnittstelle kann es sich um eine der üblichen Schnittstellen wie WLAN, Bluetooth, NFC, 3G, 4G oder 5G handeln, wobei Bluetooth 4.x oder 5.x bevorzugt wird. Das externe Darstellungsgerät ist vorzugweise ein Mobiltelefon oder eine Smart Watch.

Das externe Darstellungsgerät kann entsprechend der oben beschriebenen Darstellung das Ergebnis der Auswertung wiedergeben. Es kann darüber hinaus die Ergebnisse speichern und/oder an externe Server zur Auswertung durch Dritte weitergeben. Die Ergebnisse können auch von einem Trainingsprogramm genutzt werden, mit dem dem Benutzer die richtige Verwendung des Pumpspenders verdeutlicht wird.

Erfindungsgemäß ist vorgesehen, dass die vom Kraftsensor erfassten Daten in einem Speicher derart abgelegt werden, dass sich hieraus derzeitliche Verlauf der erfassten Kraft ergibt. Beispielsweise könnte der Kraftsensor ab der Erfassung des Beginns der Betätigung mit einer Frequenz von 500 Hz abgefragt werden und die Kraftwerte in einem Speicher abgelegt werden. Es ergibt sich hieraus der zeitliche Verlauf des Kraftwerts.

Die Auswertung, mit der ermittelt wird, ob ein für das Erreichen der betätigten zweiten Endlage charakteristischer Kraftanstieg erfolgt ist, erfolgt daher unter Berücksichtigung dieses zeitlichen Verlaufes. Eine solche Auswertung erlaubt eine höhere Zuverlässigkeit bei der Unterscheidung des Erreichens bzw. des Nicht-Erreichens der zweiten Endlage.

Die Auswertung des erfassten Kraftverlaufs über der Zeit erfolgt vorzugsweise derart, dass die Steigung des entsprechenden Graphen herangezogen wird, um die zweite Endlage zu detektieren. Insbesondere kann die Steigung der Kraft über der Zeit ausgewertet werden. Hierbei kann ein Anstieg der Steigung, also der ersten Ableitung der Kraft über der Zeit als Indikator für das Erreichen der zweiten Endlage interpretiert werden. Insbesondere kann ein Quotient aus der Steigung, also des Kraftanstiegs in einer definierten Zeitdauer, und dem Kraftwert gebildet werden. Dieser gibt die Steigung relativ zum zuvor erreichten Kraftniveau an. Dieser Quotient kann mit einem Grenzwert verglichen werden und das Überschreiten dieses Grenzwertes als Erreichen derzweiten Endlage interpretiert werden. Derentsprechende Grenzwert kann durch Versuche ermittelt werden. Insbesondere ist der Grenzwert selbst auch nicht konstant, sondern zeitlich veränderlich, nämlich absteigend. So kann er im einfachsten Falle linear absinken. Auch ist es möglich, den Grenzwert in Abhängigkeit des Erreichens vordefinierter Kraftwerte neu zu setzen.

Die Auswertung der Kraft über der Zeit erfolgt vorzugsweise anhand eines geglätteten Graphen der Kraft über der Zeit. Hierdurch werden in Art eines Tiefpassfilters hohe Frequenzen beseitigt. Solche können sich insbesondere durch Reibungan aneinander entlanggleitenden Flächen des Pumpspenders ergeben.

Die Auswertung der Kraft über der Zeit kann auch derart erfolgen, dass anhand der durch den Kraftsensor erfassten Kraft eine Kategorisierung der Betätigung in eine von mindestens zwei Kategorien erfolgt, die unterschiedlich starken und/oder schnellen Betätigungen zugeordnet sind. Diese Kategorisierung kann insbesondere in einem kurzen ersten Zeitraum der Betätigung erfolgen, beispielsweise innerhalb der ersten 0,5 Sekunden oder weniger. Die Kategorisierung gestattet es, die Erkennung, ob die zweite betätigte Endlage erreicht wurde und gegebenenfalls wann dies stattfand, für die mindestens zwei Kategorien in unterschiedlicher Weise erfolgen zu lassen. Diese unterschiedlichen Weisen können sich in verschiedenen zur Analyse herangezogenen Grenzwerten wiederspiegeln. Sie können aber auch grundsätzlich verschieden sein. So kann beispielsweise eine sehr früh vergleichsweise hohe Betätigungskraft zu einer Auswertung führen, bei der das Erreichen der zweiten Endlage als gegeben angenommen wird, da der Benutzer ein Erreichen der Endlage in der Praxis ohnehin nicht mehr verhindern kann. Ermittelt wird dann nur noch der entsprechende Zeitpunkt, wobei andere Regeln zu dessen Ermittlung herangezogen werden können als bei der langsamen oder normalen Betätigung.

Es wird als nachteilig angesehen, wenn die Auswertungseinheit zur Auswertung der Betätigungskraft vor Betätigung durch den Benutzer angeschaltet werden muss. Vorzugsweise ist die entsprechende Auswertungseinheit stattdessen stets aktiv. Um dennoch einen geringen Strombedarf zu erzielen, wird es als vorteilhaft angesehen, wenn die Auswertung unter Nutzung eines Prozessors erfolgt, der über einen normalen Betriebsmodus sowie über einen demgegenüber stromsparenden Ruhemodus verfügt. Bis zur Betätigung des Pumpspenders befindet sich der Prozessor im Ruhemodus. In diesem wird durch den Prozessor überwacht, ob der Kraftwert des Kraftsensors einen vorgegebenen Grenzwert , insbesondere vorgegeben durch ein Spannungs- oder Widerstandsniveau überschreitet. Hierfür nicht erforderliche Teile des Prozessors können derweil ausschaltet sein. Wenn der Grenzwert überschritten wird wechselt der Prozessor in den Betriebsmodus.

In diesem dann erreichten Betriebsmodus werden die vom Kraftsensor erfassten Werte mittels eines A/D-Wandlers digitalisiert und im Speicher abgelegt und anschließend vorzugsweise wiederholt vom Prozessor dahingehend analysiert, ob derfürdas Erreichen der betätigten zweiten Endlage charakteristischer Kraftanstieg zu erkennen ist. Dieser Betriebsmodus hat einen erheblich höheren Energiebedarf als der Ruhemodus. Aufgrund dem nur kurzen Verbleib im Betriebsmodus fällt dies jedoch nicht sehr ins Gewicht. Sobald ein Austragvorgang beendet wurde, entweder nach Erreichen der zweiten betätigten Endlage oder nach Nichterreichen derselben sowie nach hiermit verbundener Signalisierung, kann der Prozessor wieder in den Ruhemodus wechseln.

Neben dem beschriebenen Kraftsensor können in der zur Durchführung des Verfahrens genutzten Auswertungseinheit weitere Sensoren vorgesehen sein, so insbesondere Lagesensoren oder Beschleunigungssensoren. Deren Sensorwerte können bei dem beschriebene Verfahren ergänzend herangezogen werden, beispielsweise um eine anstehende Verwendung des Pumpspenders zu erkennen und/oder um zu überwachen, ob ein vorgesehenes Schütteln des Pumpspenders ordnungsgemäß erfolgt ist. Sensorwerte dieser Sensoren oder hieraus abgeleitete Werte können zur Beeinflussung von abgegebenen Signalen der Ausgabeeinrichtung herangezogen werden und/oder drahtlos an ein Darstellungsgerät weitergeleitet werden.

Neben dem beschriebenen Verfahren betrifft die Erfindung auch Systeme, in denen das beschriebene Verfahren Anwendung findet.

So betrifft die Erfindung insbesondere auch einen Pumpspender zum Austrag einer Flüssigkeit, insbesondere zum Austrag einer pharmazeutischen Flüssigkeit. Ein solcher Pumpspender verfügt über einen Flüssigkeitsspeicher zur Aufnahme der Flüssigkeit vor dem Austrag sowie über mindestens eine Austragöffnung, durch die Flüssigkeit in eine Umgebung abgegeben werden kann. Er verfügt weiterhin über einen Betätigungsdrücker, der gegenüber dem Flüssigkeitsspeicher verlagerbar ist, sowie über eine Pumpe mit einer Pumpkammer, wobei Wandungen der Pumpkammer zum Zwecke der Verkleinerung der Pumpkammer gegeneinander zwischen einer unbetätigten ersten Endlage und einer betätigten zweiten Endlage beweglich sind und wobei eine erste Wandung der Pumpkammer ortsfest zum Flüssigkeitsspeicher vorgesehen ist und eine zweite Wandung der Pumpkammer durch manuelle Betätigung des Betätigungsdrückers gegenüber der ersten Wandung verlagerbar ist. Die genannten Wandungen können dabei insbesondere Wandungen sein, die durch einen Pumpenzylinder und einen Pumpenkolben der Pumpe gebildet sind. Auch kann es sich um einander gegenüberliegende Wandungen einer Balgpumpe handeln.

Zusätzlich verfügt der Pumpspender über eine Auswertungseinheit zur Erkennung der Erreichung der zweiten betätigten Endlage und der Nutzung dieser Erkennung mittels des beschriebenen Verfahrens. Hierzu weist die Auswertungseinheit elektronische Komponenten auf, die vorzugsweise von einer Batterie gespeist werden. Hierzu gehört auch ein Kraftsensor im oben erläuterten Sinne, der die von einem Benutzer aufgebrachte Kraft zwischen dem Betätigungsdrückerund der zweiten Wandung, beispielsweise dem Pumpenkolben, oder zwischen einer Haltefläche am Flüssigkeitsspeicher und der ersten Wandung, beispielsweise dem Pumpenzylinder misst. Die Auswertungseinheit verfügt weiterhin über einen Prozessor, der die vom Kraftsensor erfasste Kraft auswertet, so dass ein für das Erreichen der betätigten zweiten Endlage charakteristischer Kraftanstieg erkannt werden kann. Zusätzlich verfügt die Auswertungseinheit vorzugsweise über eine Ausgabeeinrichtung, die in Abhängigkeit der Kraftauswertung ein für das Ergebnis der Kraftaufwertung charakteristisches Signal als haptisches, akustisches und/oder visuelles Signal ausgibt. Es kann sich dabei insbesondere um eine Vibrationseinrichtung oder eine LED handeln. Zusätzlich oder gegebenenfalls auch alternativ zur Ausgabeeinrichtung verfügt die Auswertungseinheit über eine drahtlose Schnittstelle, die in Abhängigkeit der Kraftauswertung ein für das Ergebnis der Kraftauswertung charakteristisches Signal an ein externes Darstellungsgerätsenden kann, um eine Verwendung oder Weiterleitung in oben beschriebenen Sinne erfolgen zu lassen.

Die beschriebene Auswertungseinheit kann fest in den Pumpspender integriert sein, so dass sie von dessen Bauteilen, insbesondere von der Pumpe, nicht werkzeuglos trennbar ist. Dies kann bei Spendern mit wechselbaren oder nachfüllbaren Flüssigkeitsspeichern zweckmäßig sein.

Bevorzugt wird allerdings die Verwendung einer ebenfalls von der Erfindung umfasst Auswertungseinheit für einen Pumpspender zum Austrag einer Flüssigkeit, insbesondere zum Austrag einer pharmazeutischen Flüssigkeit, die für eine werkzeuglose Anbringung an einem Pumpspender vorgesehen ist. Der entsprechende Pumpspender weist in diesem Falle den Flüssigkeitsspeicher zur Aufnahme der Flüssigkeit vor dem Austrag, die mindestens eine Austragöffnung, durch die Flüssigkeit in eine Umgebung abgegeben werden kann, und einen Betätigungsdrücker, der gegenüber dem Flüssigkeitsspeicher verlagerbar ist, sowie die Pumpe auf, die durch Verlagerung des Betätigungsdrückers gegenüber dem Flüssigkeitsspeicher zwischen einer unbetätigten ersten Endlage und einer betätigten zweiten Endlage betätigbar ist.

Die werkzeuglos an diesem Pumpspender anbringbare Auswertungseinheit ist zur Erkennung der Erreichung der zweiten betätigten Endlage mittels des oben beschriebenen Verfahrens ausgebildet. Die Auswertungseinheit wird vorzugsweise über einen Klemm- oder Schnappmechanismus am Pumpspender angebracht. Die Anbringung erfolgt dabei vorzugsweise derart, dass der Kraftsensor der Auswertungseinheit zwischen einer Betätigungsfläche der Auswertungseinheit und einer hierdurch bezüglich der Handhabung ersetzten und nur noch mittelbar betätigten Betätigungsfläche am Pumpspender angeordnet ist.

Bei einer ersten bevorzugten Bauform ist vorgesehen, dass die Auswertungseinheit zur Anbringung am Flüssigkeitsspeicher ausgebildet ist und über einen Befestigungsbereich zur ortsfesten Anlage an den Flüssigkeitsspeicher oder eine hierzu ortsfeste Kopplungsfläche sowie über eine Haltefläche zum manuellen Ergreifen der Auswertungseinheit verfügt. Die Auswertungseinheit verfügt weiterhin über den beschriebenen Kraftsensor, der in diesem Falle die von einem Benutzer aufgebrachte Kraft zwischen der Haltefläche und dem Befestigungsbereich erfasst. Bei einer solchen Gestaltung ist demnach vorgesehen, dass der Benutzer nicht mehr den Flüssigkeitsspeicher selbst im Bereich der Haltefläche umgreift, um die beiden Teileinheiten des Pumpspenders aufeinander zuzudrücken, sondern stattdessen die Haltefläche der Auswertungseinheit. Wenn nun die andere Teileinheit des Pumpspenders, insbesondere die genannte Betätigungseinheit mit Betätigungsdrücker niedergedrückt wird, so wirkt diese Kraft auch zwischen der Haltefläche der Auswertungseinheit und dem Befestigungsbereich und kann dort mittels des Kraftsensors erfasst werden. Der Befestigungsbereich ist dabei jener Abschnitt der Auswertungseinheit, der unmittelbar am Flüssigkeitsspeicher odereinerdazu ortsfesten Fläche anliegt und der in der Lage ist, eine in Richtung der anderen Teileinheit wirkende Kraft auf die Teileinheit des Flüssigkeitsspeichers auszuüben. Bei besonders bevorzugten Ausgestaltungen handelt es sich bei dem Befestigungsbereich nicht nur um eine Anlagefläche zur Kraftbeaufschlagung des Flüssigkeitsspeichers, sondern um eine Haltevorrichtung, die derart mit der Teileinheit des Flüssigkeitsspeichers verkoppelt ist, dass der Flüssigkeitsspeicher hieran ausreichend fest gehalten ist, dass er sich selbst sin einer Überkopflage nicht vom Befestigungsbereich trennt.

Insbesondere vorzugsweise weist eine zur Anbringung am Flüssigkeitsspeicher ausgebildete Auswertungseinheit ein Gehäuse mit einer Mantelwandung auf, in die der Flüssigkeitsspeicher des Pumpspenders einschiebbar ist und deren Außenseite die Haltefläche bildet. Der Befestigungsbereich der Auswertungseinheit ist dabei vorzugsweise innerhalb der Mantelwandung vorgesehen und kommt dort mit dem eingeschobenen Pumpspender in Kontakt.

Der Befestigungsbereich ist vorzugsweise mit mindestens einem elastisch auslenkbaren Halteelement versehen und derart zur Aufnahme des Pumpspenders ausgebildet, dass bei in den Befestigungsbereich eingeschobenem Pumpspender das mindestens eine Halteelement elastisch ausgelenkt ist und hierdurch eine Haltekraft bewirkt. Insbesondere kann der Befestigungsbereich durch einen Befestigungsbecher gebildet werden, der umfänglich verteilt mehrere elastisch auslenkbare Halteelemente aufweist, beispielsweise in Form von nach innen weisenden Rippen an einer umlaufenden Ringwandung des Befestigungsbechers.

Bei einer alternativen zweiten Bauform ist die Auswertungseinheit an der anderen Teileinheit vorgesehen. Die Auswertungseinheit ist dabei also zur Anbringung am Betätigungsdrücker oder der Teileinheit des Betätigungsdrückers ausgebildet und verfügt in diesem Fall über einen Befestigungsbereich zur ortsfesten Anlage an den Betätigungsdrücker sowie über eine Betätigungsfläche zum manuellen Niederdrücken der Auswertungseinheit. Wie auch bei der ersten dargestellten Bauform erfolgt die Befestigung dabei vorzugsweise mittels einer Klemm- oder Schnappverbindung. Die Auswertungseinheit verfügt ebenfalls über einen Kraftsensor im eingangs beschriebenen Sinne, der in diesem Falle die von einem Benutzer aufgebrachte Kraft zwischen der Betätigungsfläche und dem Befestigungsbereich erfasst. Bei dieser alternativen Gestaltung ist vorgesehen, dass der Benutzer nicht mehr unmittelbar den am Pumpspender vorgesehenen Betätigungsdrücker niederdrückt, um die beiden Teileinheiten des Pumpspenders aufeinander zuzudrücken, sondern stattdessen die Betätigungsfläche der Auswertungseinheit. Wenn nun die Betätigungsfläche gegenüber der anderen Teileinheit des Pumpspenders niedergedrückt wird, so wirkt diese Kraft auch zwischen der Betätigungsfläche der Auswertungseinheit einerseits und dem Betätigungsdrücker des Pumpspenders und dem daran anliegenden Befestigungsbereich andererseits und kann dort mittels des Kraftsensors erfasst werden.

Unabhängig davon, ob die Auswertungseinheit am Flüssigkeitsspeicher oder an der Betätigungseinheit angebracht ist, verfügt sie über einen Prozessor, der die vom Kraftsensor erfasste Kraft auswertet, so dass ein für das Erreichen der betätigten zweiten Endlage charakteristischer Kraftanstieg erkannt wird, sowie über eine Ausgabeeinrichtung, die in Abhängigkeit der Kraftauswertung ein für das Ergebnis der Kraftauswertung charakteristisches Signal als haptisches, akustisches und/oder visuelles Signal ausgibt und/oder über eine drahtlose Schnittstelle, die in Abhängigkeit der Kraftauswertung ein für das Ergebnis der Kraftauswertung charakteristisches Signal an ein externes Darstellungsgerät senden kann.

Eine Auswertungseinheit zur werkzeuglosen Anbringung gestattet es, einfach an einen Pumpspender angekoppelt zu werden und von diesem wieder entkoppelt zu werden, wenn der Flüssigkeitsspeicher entleert ist und die Auswertungseinheit an einem neuen Pumpspender angebracht werden soll.

Die genannte werkzeuglos anbringbare Auswertungseinheit wird bestimmungsgemäß zusammen mit einem Pumpspender verwendet und kann auch als Teil eines Sets aus einem Pumpspender und einer Auswertungseinheit angeboten werden.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A und 1B zeigen einen Pumpspender, der beim vorliegenden Beispiel als Pumpspender zur sublingualen Applikation einer pharmazeutischen Flüssigkeit ausgebildet ist.
Fig. 2 zeigt den Spender der Fig. 1A und 1B, der um eine werkzeuglos angekoppelte Auswertungseinheit ergänzt ist.
Fig. 3 zeigt die Einzelkomponenten der Auswertungseinheit der Fig. 2.
Fig 4A bis 4C verdeutlichen die Verwendung des Pumpspenders mit angebrachter Auswertungseinheit.
Fig. 5A bis 5C zeigen Kraft-Zeit-Verläufe für unterschiedliche Arten der Betätigung.
Fig. 6 zeigt eine alternative Ausgestaltung einer am Pumpspender der Fig. 1A und 1B angebrachten Auswertungseinheit.

### DETAILLIERTE BESCHREIBUNG DER AUSFÜHRUNGSBEISPIELE

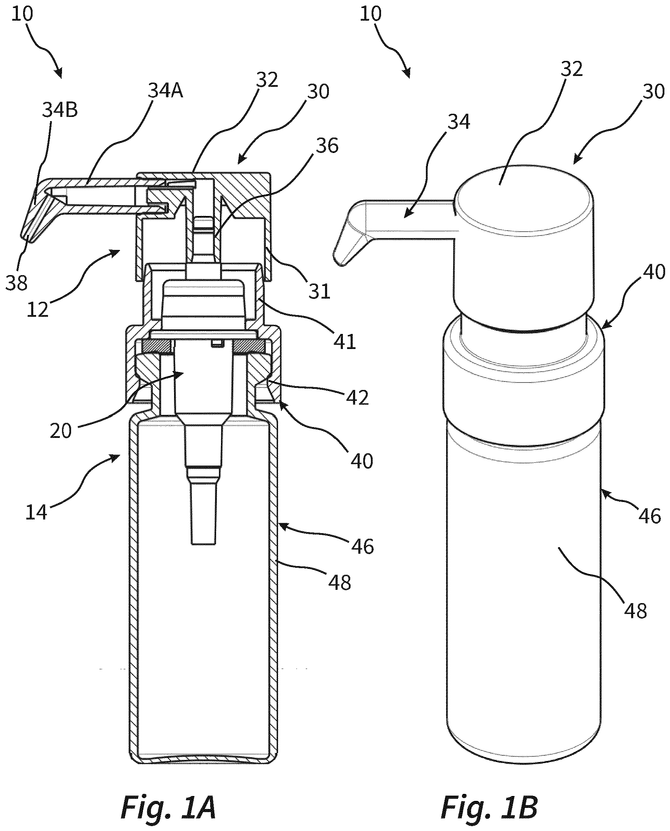

Die Fig. 1A und 1B zeigen einen Pumpspender 10 von an sich bekannter Bauweise. Der Pumpspender 10 umfasst einen Flüssigkeitsspeicher46, in dem eine pharmazeutische Flüssigkeit vor dem Austrag gelagert ist. Zum Austrag dieser Flüssigkeit verfügt der Pumpspender 10 über eine Pumpe 20 und eine Austragöffnung 38. Die Austragöffnung 38 ist beim dargestellten Pumpspender 10 in Hinblick auf den bestimmungsgemäßen Austrag von Flüssigkeit unter der Zunge des Benutzers am distalen Ende eines seitlich frei hervor ragenden Applikatorrohres 34 vorgesehen, welches bei der konkreten Gestaltung einen radialen Rohrabschnitt 34A und eine davon nach unten abknickende Applikatorrohrspitze 34B aufweist.

Die Austragöffnung 38 ist an einer Betätigungseinheit 30 vorgesehen, die gegenüber dem Flüssigkeitsspeicher 46 und einer hiermit verbundenen Basis 40 in einer Vertikalrichtung niederdrückbar ist, wobei die Betätigungseinheit 30 dabei durch einen an einer Gleitfläche 41 der Basis 40 gleitenden Gleitring 31 geführt ist. Das Niederdrücken erfolgt bestimmungsgemäß dadurch, dass der Benutzer den Flüssigkeitsspeicher 46 mit der Hand im Bereich der Haltefläche 48 und/oder der Basis 40 umgreift und mit seinem Zeigefinger die Betätigungseinheit 30 an deren Betätigungsdrücker 32 nach unten kraftbeaufschlagt. Hierdurch wird eine Pumpe 20 betätigt, deren Pumpzylinder zwischen dem Flaschenkörper des Flüssigkeitsspeichers 46 und der Basis 40 eingeklemmt ist, während eine nach oben aus dem Pumpzylinder hinausragende Kolbeneinheit mit Pumpausgangskanal in einen Austragkanal36 der Betätigungseinheit klemmend eingeschoben ist. Durch das Niederdrücken der Betätigungseinheit 30 gegenüber dem Flüssigkeitsspeicher 46 wird die Kolbeneinheit in den Pumpenzylinder gedrückt und die in der hierdurch begrenzten Pumpkammer enthaltene Flüssigkeit zur Austragöffnung gefördert. Das bestimmungsgemäße Austragvolumen wird dabei nur dann erreicht, wenn die Betätigungseinheit 30 bis in ihre mechanisch vorgegebene Endlage gedrückt wird.

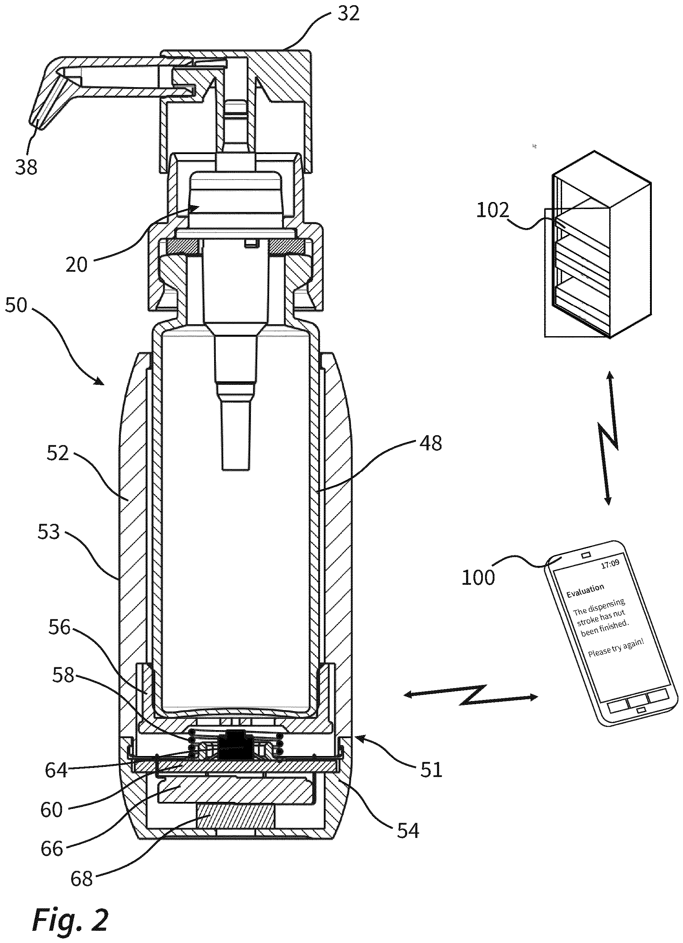

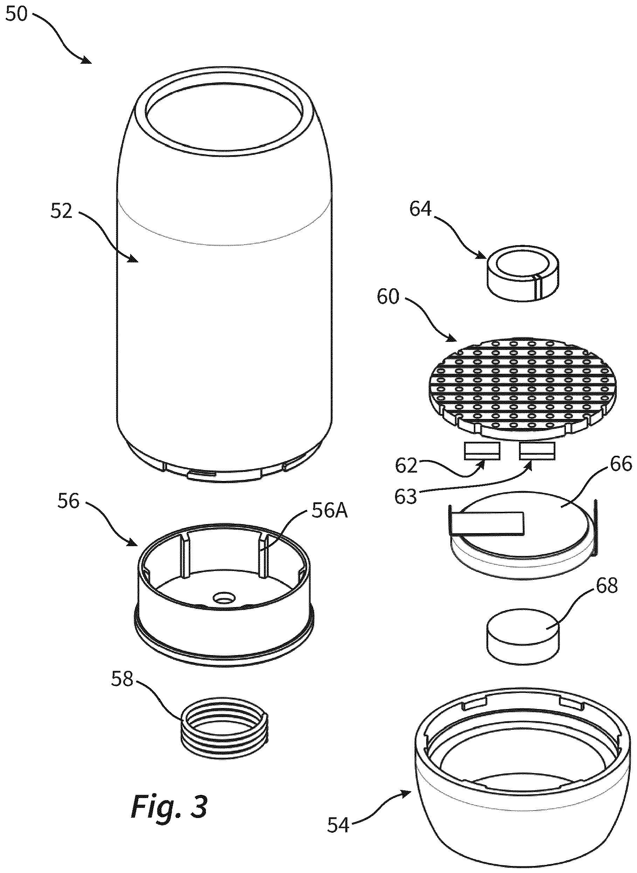

Zur Überwachung dessen ist die Auswertungseinheit 50 vorgesehen, die in Fig. 2 in geschnittener Darstellung gezeigt ist und deren Einzelkomponenten der Explosionsdarstellung der Fig. 3 entnommen werden können. Die Auswertungseinheit umfasst ein aus zwei Bauteilen 52, 54 gebildetes Gehäuse, wobei das Bauteil 54 einen Boden und das Bauteil 52 eine Mantelwandung bildet, die einen Aufnahmeraum umgibt. Dieser Aufnahmeraum ist an seinem unteren Ende durch ein einen Befestigungsbereich 56 bildendes becherartiges Bauteil begrenzt, in welches der Flüssigkeitsspeicher 46 unter Bildung einer Klemmverbindung eingeschoben ist. Innenseitige Rippen 56A des Bauteils werden beim Einschieben des Flüssigkeitsspeichers 46 nach außen ausgelenkt und verursachen so eine Klemmspannung. Unterhalb des becherartigen Bauteils ist eine Platine 60 vorgesehen, auf deren Oberseite ein Kraftsensor 64 in Art eines FSR-Sensors vorgesehen ist. Dieser ist umgeben von einer Druckfeder58, die im vollständig entspannten Zustand das becherartige Bauteil derart anhebt, dass es den Berührkontakt zum Kraftsensor 64 verliert. Auf der Unterseite der Platine 60 sind ein Prozessor 62 sowie ein Funkmodul 63 zur Kommunikation mit einem Mobiltelefon 100 und mittelbar mit einem Server 102 vorgesehen. Weiterhin sind eine Batterie 66 sowie ein Vibrationssignalgeber 68 mit Kontakten an der Unterseite der Platine 60 verbunden.

Der um die Auswertungseinheit 50 ergänzte Pumpspender 10 ist für den Benutzer in nahezu unveränderter Art bedienbar. Allerdings umgreift der Benutzer mit seiner Hand nunmehr nicht mehr unmittelbar die Haltefläche 48 des Flüssigkeitsspeichers 46, sondern die Haltefläche 53 der Auswertungseinheit 50. Drückt er dann auf den Betätigungsdrücker 32, so wirkt die entsprechende Kraft unter Berücksichtigung der Federkraft der Feder 58 auch auf den Kraftsensor 64, so dass dem Prozessor 62 die entsprechenden Kraftsensorwerte zur Verfügung stehen.

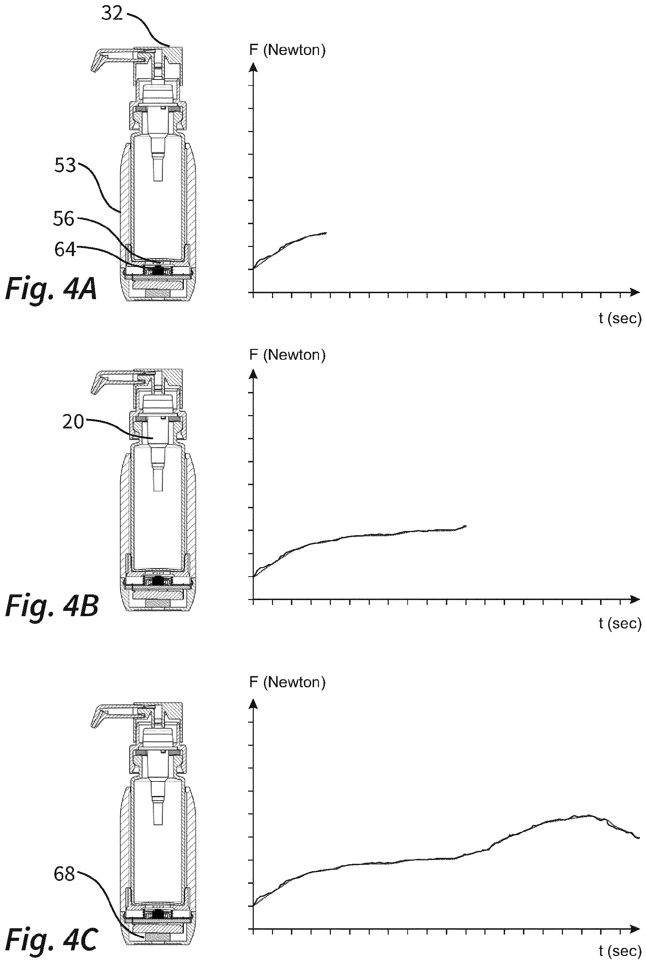

Die Fig. 4A bis 4C erläutern die Erfassung der Kraft während der Betätigung.

Vor der Benutzung befindet sich der Prozessor 62 in einem stromsparenden Modus, in welchem er verbleibt, bis erstmals der Kraftsensorwert des Kraftsensors 64 den Wert Null übersteigt. Dies ist der Fall, sobald bei der Betätigung die Feder 58 soweit komprimiert ist, dass der Kraftsensor 64 vom Befestigungsbereich 56 berührt und kraftbeaufschlagt wird.

Zu diesem Zeitpunkt wechselt der Prozessor 62 in seinen Betriebsmodus und speichert in diesem die Kraftsensorwerte des Kraftsensors 64 in einer vorgegebenen Frequenz, beispielsweise 500 Hz ab. Im Speicher der Auswertungseinheit 50 werden ab diesem Zeitpunkt die entsprechenden Werte abgelegt. Die in den Fig. 4A bis 4C rechts dargestellten Diagramme verdeutlichen dies anhand derjeweils durchgezogenen Linie.

In einer ersten Betätigungsphase, gegen Ende derer der Zustand der Fig. 4A erreicht ist, steigt der Kraftsensorwert an. Dies ist die Phase vor dem Austrag, in welchem zunächst die Spalte zwischen Einzelkomponenten des Pumpspenders und auch zur Auswertungseinheit zusammengedrückt werden. Gegen Ende dieser ersten Betätigungsphase erfolgt der Flüssigkeitsaustrag.

Fig. 4B verdeutlicht, dass die Betätigungskraft während des Flüssigkeitsaustrags nur leicht ansteigt. Dieser Anstieg ist primär durch die sich zunehmend spannende Feder der Pumpe 20 bedingt. Die Figur 4B zeigt den Pumpspender 10 am Ende der Phase des Flüssigkeitsaustrags. Der Pumpenkolben hat nun seine durch die Geometrie der Pumpenkammer definierte Endlage erreicht.

Fig. 4C verdeutlicht, dass nun ein weiterer Kraftanstieg folgt, während die Relativlage der Teileinheiten des Pumpenspenders sich nur noch geringfügig ändert. Dies ist durch die Verformbarkeit der Bauteile bedingt, führt jedoch nicht mehrzu einem Flüssigkeitsaustrag. Der Benutzer kann das Ende des Flüssigkeitsaustrags sowie die ansteigende Kraft bemerken.

Insbesondere jedoch bemerkt er eine Vibration, die durch den Vibrationssignalgeber 68 erzeugt wird, da der Prozessor 62 anhand des in den Figuren 4A bis 4C dargestellten Verlaufs erkannt hat, dass die zweite betätigte Hubendlage erreicht wurde.

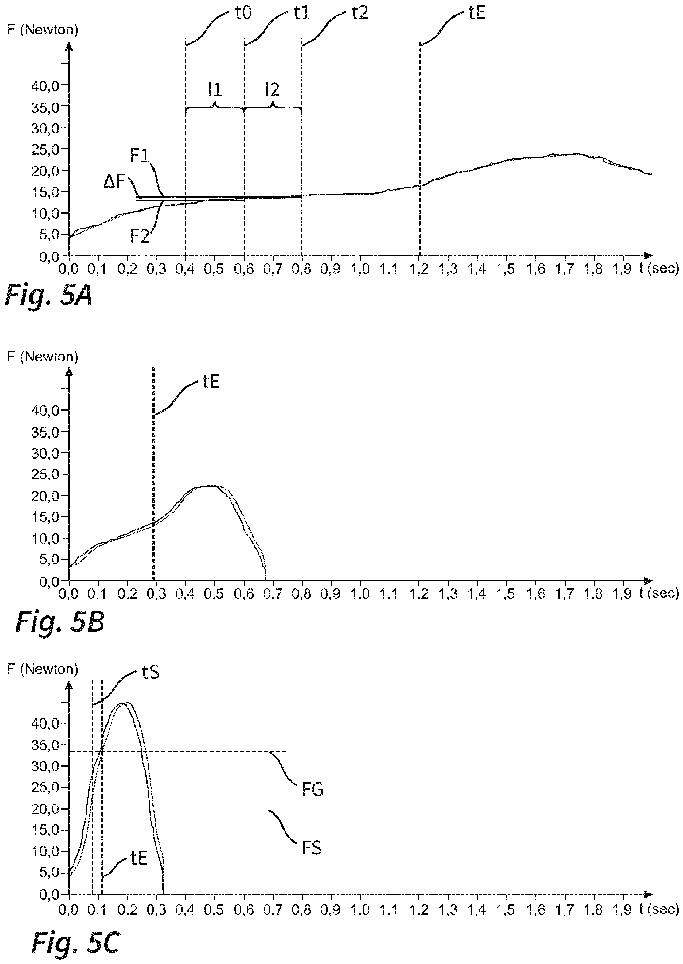

Anhand der Fig 5A bis 5C wird erläutert, wie die Auswertung durch den Prozessor 62 erfolgen kann. Dabei zeigt die Fig. 5A den Kraft-Zeit-Verlauf entsprechend den Figuren 4A bis 4C. Die Diagramme der Fig. 5B und 5C zeigen jeweils in unterschiedlichem Maße kraftvollere Betätigungen.

Die Auswertung erfolgt wie folgt:
Fig. 5A zeigt ebenso wie die Fig. 4A bis 4C den mittels des Kraftsensors ermittelten Kraft-Zeit-Verlauf mit einer durchgezogenen Linie. Mittels des Prozessors 62 wird dieser Verlauf geglättet, beispielsweise mittels der Methode des gleitenden Mittelwertes. Der entsprechend geglättete Verlauf ist in den Figuren mit einer gepunkteten Linie verdeutlicht.

Dieser geglättete Verlauf wird primär zur weiteren Analyse herangezogen. Eine mögliche Methode sieht dabei vor, dass der Prozessor die bereits vorliegenden geglätteten Messwerte dahingehend nutzt, dass er zu einem aktuellen Zeitpunkt t2 für Zeitpunkte der Vergangenheit, exemplarisch hier für den Zeitpunktt1, jeweils ein von diesem Zeitpunkt aus zurückliegendes Zeitintervall I1 sowie ein seinerzeit in der Zukunft liegendes Zeitintervall I2 untersucht. Für beide Intervalle definierter zeitlicher Länge, wird ein mittlerer Kraftwert ermittelt, die hier F1 und F2 genannt werden. Die Differenz der mittleren Kraftwerte wird hier ΔF genannt. Aus der Kraftdifferenz ΔF und einem der beiden Werte F1 und F2 oder ihre Summe wird ein Quotient gebildet, also beispielsweise ΔF/F1, der gleichsam angibt, wie stark die Kurve der Messwerte im Intervall I2 in Relation zum vorherigen Intervall I1 ansteigt.

Dieser Quotient, vorliegend exemplarisch ΔF/F1, wird mit einem Grenzwert verglichen. Der Grenzwert ist dabei im Regelfall ein zeitlich veränderlicher Grenzwert, der ausgehend vom Beginn der manuellen Kraftbeaufschlagung über die Zeit sinkt, im einfachsten Falle linear. Liegt der Quotient oberhalb des Grenzwertes, so wird dies als Erreichen der zweiten betätigten Endlage interpretiert. Im Falle der Betätigung der Fig. 5A ist ein Überschreiten des Grenzwertes zum Zeitpunkt tE gegeben. Der Grenzwert kann alternativ zu einem Absinken über die Zeit auch stufenweise eine Anpassung erfahren, wenn jeweils ein zugeordneter Kraftwert erreicht wurde.

Das beschriebene Verfahren gestattet die korrekte Interpretation der Kraftverläufe, wie sie sich bei langsamer und normaler Betätigung entsprechend den Figuren 5A und 5B ergeben.

Es hat sich allerdings gezeigt, dass sehr kraftvolle Betätigungen, wie in Figur 5C dargestellt, mit diesem Vorgehen nicht immer sehr gut zu analysieren sind, da hier die Kraft über der Zeit nach Erreichen der betätigten Endlage nicht mehr in gleichem Maße wie bei normaler oder langsamer Betätigung ansteigt.

Das beschriebene Verfahren ist daher um folgende Vorgehensweise ergänzt: Wird nach Beginn der Betätigung ein sofortiger deutlicher Anstieg der gemessenen Kraft verzeichnet, also beispielsweise ein Erreichen oder Überschreiten eines vorgegebenen Kraftwertes FS innerhalb eines kurzen Zeitraum tS, oder wird mittels einer anderen Art der Berechnung eine frühe starke Steigung des Graphen ermittelt, so ist die ein Indikator für eine sehr kraftvolle und/oder schnelle Betätigung. Bei einer solchen Betätigung ist das Erreichen der zweiten Endlage durch den Benutzer in der Praxis nicht mehr zu verhindern. Es wird also bereits innerhalb des kurzen Zeitraums tS klar, dass die zweite Endlage ordnungsgemäß erreicht wird. In diesem Falle wird der Zeitpunkt des Erreichens der zweiten betätigten Endlage nicht mehr anhand einer Auswertung der Steigung wie oben beschrieben ermittelt, sondern anhand des Erreichens einer Grenzkraft FG, die oberhalb des Kraftwertes FS liegt. Überschreitet der gemessene Kraftwert diese Grenzkraft FG, so ist dies ein ausreichend genauer Indikator für das Erreichen der zweiten Endlage.

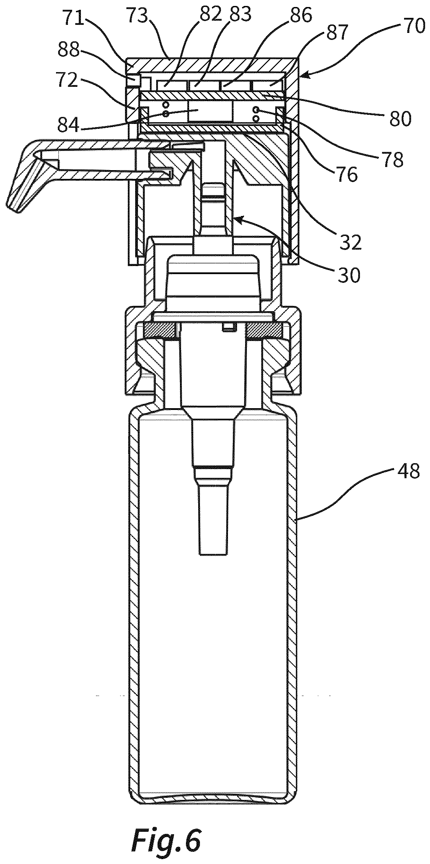

Fig. 6 verdeutlicht in schematischer Darstellung eine alternative Bauweise einer Auswertungseinheit 70. Diese ist zum Aufsetzen auf die Betätigungseinheit 30 und den dortigen Betätigungsdrücker 32 vorgesehen. Sie umfasst ähnliche Komponenten wie die Auswertungseinheit 50, nämlich ein Gehäuse 71, welches statt der Haltefläche 53 eine oberseitige Betätigungsfläche 73 aufweist. Das Gehäuse 71 weist eine Mantelfläche auf, die im Bereich des Applikatorrohres 34 eine Aussparung aufweist. Am unteren Ende der Mantelfläche ist eine Rastnocke zur Ankopplung an die Betätigungseinheit 30, vorliegend an deren Gleitring 31, vorgesehen. Innerhalb des Gehäuses 71 ist der Befestigungsbereich 76 zur ortsfesten Anlage an den Betätigungsdrücker 32 vorgesehen. Weiterhin ist hier ähnlich wie bei der Auswertungseinheit 50 eine Platine 80 vorgesehen, bestückt mit einem Kraftsensor 84, einem Mikroprozessor 82, einem Funkmodul 83 sowie einer Batterie 86. Als Ausgabeeinrichtung ist statt des Vibrationssignalgebers 68 eine LED 88 vorgesehen. Exemplarisch für beiden Auswertungseinheiten 50, 70 weist die Auswertungseinheit 70 darüber hinaus einen Lage- und Beschleunigungssensor 87 auf, mittels dessen die vertikale Ausrichtung des Spenders bei Betätigung und/oder das ordnungsgemäße Schütteln vor der Betätigung erfasst werden kann.

Der Kraftsensor 84 erfasst ebenso wie der Kraftsensor 64 die Betätigungskraft. Grundsätzlich stellt sich daher auch ein identischer Kraft-Zeit-Verlauf ein, wie er in den Figuren 4A bis 4C und 5A bis 5C verdeutlicht ist. Abweichend von der Beschreibung hierzu ist bei der vorliegenden Gestaltung vorzugsweise vorgesehen, dass bereits der Beginn der Betätigung und somit der Auswertung signalisiert wird, beispielsweise indem die LED gelb strahlt. Wird dann der für die zweite betätigte Endlage der Pumpe 20 nicht erkannt, beispielsweise für 1 Sekunde, so wechselt die Farbe auf Rot. Wird dagegen die zweite betätigte Endlage erreicht und somit ein vollständiger ordnungsgemäßer Austrag erkannt, so wechselt die Farbe auf grün.

## Patentansprüche

1. Verfahren zur Auswertung einer Pumpbetätigung an einem Pumpspender (10) zum Austrag einer Flüssigkeit, insbesondere einer pharmazeutischen Flüssigkeit, mit den folgenden Merkmalen:
a. das Verfahren findet Anwendung an einem Pumpspender (10), der über zwei gegeneinander manuell bewegbare Teileinheiten (12, 14) verfügt und der über eine Pumpe (20) mit einerdurch die Bewegung der Teileinheiten (12, 14) von einer unbetätigten ersten Endlage in eine betätigte zweite Endlage verkleinerbare Pumpkammer verfügt, und
b. zur Erfassung der betätigten zweiten Endlage wird während der Bewegung der Teileinheiten (12, 14) zueinander mittels eines Kraftsensors (64, 84) eine Kraft erfasst, mit der die Teileinheiten (12, 14) manuell aufeinander zu bewegt werden, und
c. es wird der Verlauf der erfassten Kraft über die Zeit dahingehend ausgewertet, dass ein für das Erreichen der betätigten zweiten Endlage charakteristischer Kraftanstieg erkannt wird, und
d. in Abhängigkeit davon, ob der für das Erreichen der betätigten zweiten Endlage charakteristischer Kraftanstieg erfasst wurde, wird ein für das Ergebnis charakteristisches Signal durch eine Ausgabeeinrichtung haptisch, akustisch und/oder visuell ausgegeben und/oder über eine drahtlose Schnittstelle an ein externes Darstellungsgerät gesendet,
**gekennzeichnet durch** die folgenden weiteren Merkmale:
e. die vom Kraftsensor (64, 84) erfassten Daten werden in einem Speicher derart abgelegt, dass sich hieraus der zeitliche Verlauf der erfassten Kraft ergibt, und
f. die Auswertung, mit der ermittelt wird, ob ein für das Erreichen der betätigten zweiten Endlage charakteristischer Kraftanstieg erfolgt ist, erfolgt unter Berücksichtigung des erfassten zeitlichen Verlaufes.

2. Verfahren nach Anspruch 1, **gekennzeichnet durch** folgende weitere Merkmale:
a. die Auswertung erfolgt unter Nutzung eines Prozessors (62, 82), der über einen Betriebsmodus sowie über einen demgegenüber stromsparenden Ruhemodus verfügt, und
b. der Kraftsensor (64, 84) ist derart mit dem Prozessor verbunden, dass der Prozessor (62, 82) im Ruhemodus nach Überschreiten eines vorgegebenen Grenzwertes in den Betriebsmodus wechselt, und
c. anschließend werden im Betriebsmodus die vom Kraftsensor (64, 84) erfassten Werte im Speicher abgelegt und wiederholt vom Prozessor (62, 82) analysiert, um den für das Erreichen der betätigten zweiten Endlage charakteristischer Kraftanstieg zu erkennen.

3. Verfahren nach Anspruch 1 oder 2, **gekennzeichnet durch** mindestens eines der folgenden weiteren Merkmale:
a. die Auswertung der Kraft über der Zeit erfolgt anhand eines geglätteten Graphen der Kraft über der Zeit,
und/oder
b. die Auswertung der Kraft über der Zeit erfolgt derart, dass die Steigung der Kraft über der Zeit ausgewertet wird, wobei ein Anstieg der Steigung als Indikator für das Erreichen der zweiten Endlage interpretiert wird und wobei insbesondere ein Quotient aus der Steigung und dem Kraftwert mit einem Grenzwert verglichen wird und das Überschreiten dieses Grenzwertes als Erreichen der zweiten Endlage interpretiert wird, wobei insbesondere der Grenzwert zeitabhängig oder kraftabhängig ist und über die Zeit sinkt,
und/oder
c. die Auswertung der Kraft über der Zeit erfolgt derart, dass anhand der durch den Kraftsensor erfassten Kraft eine Kategorisierung der Betätigung in eine von mindestens zwei Kategorien erfolgt, die unterschiedlich starken und/oder schnellen Betätigungen zugeordnet sind, wobei die Erkennung des Erreichens der zweiten betätigten Endlage und/oder die Erkennung des Zeitpunktes des Erreichens der zweiten betätigten Endlage für die mindestens zwei Kategorien in unterschiedlicher Weise erfolgt.

4. Verfahren nach einem der vorstehenden Ansprüche, **gekennzeichnet durch** mindestens eines der folgenden weiteren Merkmale:
a. die Signalisierung erfolgt derart, dass nach Beginn der Betätigung ein erstes Signal ausgegeben wird, welches endet, sobald der für das Erreichen der betätigten zweiten Endlage charakteristischer Kraftanstieg erkannt wird und/oder sobald ein für ein Ende der Betätigung charakteristischer Kraftverlauf oder Kraftwert gemessen wurde, und
b. die Signalisierung erfolgt derart, dass ein zweites Signal ausgegeben wird, wenn der für das Erreichen der betätigten zweiten Endlage charakteristischer Kraftanstieg erkannt wurde, und
c. die Signalisierung erfolgt derart, dass ein drittes Signal ausgegeben wird, sobald ein für ein Ende der Betätigung charakteristischer Kraftverlauf oder Kraftwert gemessen wurde.

5. Verfahren nach einem der vorstehenden Ansprüche, **gekennzeichnet durch** folgendes weiteres Merkmal:
a. das Verfahren sieht vor, dass die mittels des Kraftsensors (64, 84) erfassten Messdaten und/oder Daten zur Erfassung des Kraftanstiegs über die drahtlose Schnittstelle (63, 83) an das externe Darstellungsgerät (100) gesendet werden,
insbesondere mit mindestens einem der folgenden zusätzlichen Merkmale:
b. auf dem externen Darstellungsgerät (100) wird dem Benutzer angezeigt, ob die zweite betätigte Endlage beim letzten Austrag erzielt wurde, und/oder
c. das Darstellungsgerät sendet die erfassten Messdaten und/oder Daten zur Erfassung oder Nichterfassung des für das Erreichen der zweiten betätigten Endlage charakteristischen Kraftanstiegs an einen externen Server (102), insbesondere zur Übermittlung an einen Arzt.

6. Pumpspender (10) zum Austrag einer Flüssigkeit, insbesondere zum Austrag einer pharmazeutischen Flüssigkeit, mit den folgenden Merkmalen
a. der Pumpspender (10) verfügt über einen Flüssigkeitsspeicher (46) zur Aufnahme der Flüssigkeit vor dem Austrag sowie über mindestens eine Austragöffnung (38), durch die Flüssigkeit in eine Umgebung abgegeben werden kann, und
b. der Pumpspender (10) verfügt über einen Betätigungsdrücker (32), der gegenüber dem Flüssigkeitsspeicher (46) verlagerbar ist,
c. der Pumpspender (10) verfügt über eine Pumpe (20) mit einer Pumpkammer, wobei Wandungen der Pumpkammer zum Zwecke der Verkleinerung der Pumpkammer gegeneinander zwischen einer unbetätigten ersten Endlage und einer betätigten zweiten Endlage beweglich sind und wobei eine erste Wandung der Pumpkammer ortsfest zum Flüssigkeitsspeicher (46) vorgesehen ist und eine zweite Wandung der Pumpkammer durch manuelle Betätigung des Betätigungsdrückers (32) gegenüber der ersten Wandung verlagerbar ist, und
d. der Pumpspender (10) verfügt über eine Auswertungseinheit (50, 70) zur Erkennung der Erreichung der zweiten betätigten Endlage mittels des Verfahrens gemäß einem der Ansprüche 1 bis 5, wobei
- die Auswertungseinheit (50, 70) über einen Kraftsensor (64, 84) verfügt, der die von einem Benutzer aufgebrachte Kraft zwischen dem Betätigungsdrücker (32) und der zweiten Wandung oder zwischen einer Haltefläche (48) am Flüssigkeitsspeicher (46) und der ersten Wandung misst, und
- die Auswertungseinheit (50, 70) über einen Prozessor (62, 82) verfügt, der die vom Kraftsensor (64, 84) erfasste Kraft auswertet, so dass ein für das Erreichen der betätigten zweiten Endlage charakteristischer Kraftanstieg erkannt wird, und
- die Auswertungseinheit (50, 70) über eine Ausgabeeinrichtung (68, 88) verfügt, die in Abhängigkeit der Kraftauswertung ein für das Ergebnis der Kraftaufwertung charakteristisches Signal als haptisches, akustisches und/oder visuelles Signal ausgibt und/oder die Auswertungseinheit (50, 70) über eine drahtlose Schnittstelle (63, 83) verfügt, die in Abhängigkeit der Kraftauswertung ein für das Ergebnis der Kraftaufwertung charakteristisches Signal an ein externes Darstellungsgerät (100) senden kann.

7. Auswertungseinheit (50, 70) für einen Pumpspender zum Austrag einer Flüssigkeit, insbesondere zum Austrag einer pharmazeutischen Flüssigkeit, mit den folgenden Merkmalen:
a. die Auswertungseinheit (50,70) ist für eine werkzeuglose Anbringung an einem Pumpspender (10) vorgesehen, der über einen Flüssigkeitsspeicher (46) zur Aufnahme der Flüssigkeit vor dem Austrag, über mindestens eine Austragöffnung (38), durch die Flüssigkeit in eine Umgebung abgegeben werden kann, und über einen Betätigungsdrücker (32), der gegenüber dem Flüssigkeitsspeicher (46) verlagerbar ist, verfügt, wobei der Pumpspender (10) weiterhin über eine Pumpe (20) verfügt, die durch Verlagerung des Betätigungsdrückers (32) gegenüber dem Flüssigkeitsspeicher (46) zwischen einer unbetätigten ersten Endlage und einer betätigten zweiten Endlage betätigbar ist, und
b. die Auswertungseinheit (50, 70) ist zur Erkennung der Erreichung der zweiten betätigten Endlage mittels des Verfahrens gemäß einem der Ansprüche 1 bis 5 ausgebildet, wobei
- die Auswertungseinheit (50) ist zur Anbringung am Flüssigkeitsspeicher (46) ausgebildet und verfügt über einen Befestigungsbereich (56) zur ortsfesten Anlage an den Flüssigkeitsspeicher (46) sowie über eine Haltefläche (53) zum manuellen Ergreifen der Auswertungseinheit (50), wobei die Auswertungseinheit weiterhin über einen Kraftsensor (64) verfügt, der die von einem Benutzer aufgebrachte Kraft zwischen der Haltefläche (53) und dem Befestigungsbereich (56) erfasst,
oder
die Auswertungseinheit (70) ist zur Anbringung am Betätigungsdrücker (32) ausgebildet und verfügt über einen Befestigungsbereich (76) zur ortsfesten Anlage an den Betätigungsdrücker (32) sowie über eine Betätigungsfläche (73) zum manuellen Niederdrücken der Auswertungseinheit (70), wobei die Auswertungseinheit (70) weiterhin über einen Kraftsensor (84) verfügt, der die von einem Benutzer aufgebrachte Kraft zwischen der Betätigungsfläche (73) und dem Befestigungsbereich (76) erfasst,
und
- die Auswertungseinheit (50, 70) über einen Prozessor (62, 82) verfügt, der die vom Kraftsensor (64, 84) erfasste Kraft auswertet, so dass ein für das Erreichen der betätigten zweiten Endlage charakteristischer Kraftanstieg erkannt wird, und
- die Auswertungseinheit (50, 70) verfügt über eine Ausgabeeinrichtung (68, 88), die in Abhängigkeit der Kraftauswertung ein für das Ergebnis der Kraftauswertung charakteristisches Signal als haptisches, akustisches und/oder visuelles Signal ausgibt und/oder die Auswertungseinheit verfügt über eine drahtlose Schnittstelle (63, 83), die in Abhängigkeit der Kraftauswertung ein für das Ergebnis der Kraftauswertung charakteristisches Signal an ein externes Darstellungsgerät (100) senden kann.

8. Auswertungseinheit (50) nach Anspruch 7 mit den folgenden weiteren Merkmalen:
a. die Auswertungseinheit (50) ist zur Anbringung am Flüssigkeitsspeicher (46) ausgebildet, und
b. die Auswertungseinheit weist ein Gehäuse (51) mit einer Mantelwandung (52) auf, in die der Flüssigkeitsspeicher (46) des Pumpspenders (10) einschiebbar ist und deren Außenseite die Haltefläche (53) bildet, und
c. der Befestigungsbereich (56) der Auswertungseinheit ist innerhalb der Mantelwandung (52) vorgesehen und kommt dort mit dem eingeschobenen Pumpspender (10) in Kontakt.

9. Auswertungseinheit (50) nach Anspruch 8 mit dem folgenden weiteren Merkmal:
d. der Befestigungsbereich (56) ist mit mindestens einem elastisch auslenkbaren Halteelement (56A) versehen und derart zur Aufnahme des Pumpspenders (10) ausgebildet, dass bei in den Befestigungsbereich (56) eingeschobenem Pumpspender (10) das Halteelement (56A) elastisch ausgelenkt ist und hierdurch eine Haltekraft bewirkt.

10. Set aus einem Pumpspender und einer Auswertungseinheit mit den folgenden Merkmalen:
a. das Set verfügt über einen Pumpspender (10) mit einem Flüssigkeitsspeicher (46) und einem gegenüber dem Flüssigkeitsspeicher (46) beweglichen Betätigungsdrücker (32), und
b. das Set verfügt über eine Auswertungseinheit (50, 70) nach einem der Ansprüche 7 bis 9 zur Anbringung am Flüssigkeitsspeicher (46) oder am Betätigungsdrücker (32) des Pumpspenders (10).

11. Pumpspender (10) nach Anspruch 6 oder Set nach Anspruch 10, **gekennzeichnet durch** das zusätzliche Merkmal:
a. der Pumpspender (10) verfügt über eine Betätigungseinheit (30), die den Betätigungsdrücker (32) und die Austragöffnung (38) umfasst und die als Ganzes zum Zwecke der Betätigung der Pumpe (20) gegenüber dem Flüssigkeitsspeicher (46) in einer Betätigungsrichtung verlagerbar ist,
insbesondere mit mindestens einem der zusätzlichen Merkmale:
b. die Austragöffnung (38) ist seitlich an der Betätigungseinheit (30) vorgesehen, so dass eine Austragrichtung mit der Betätigungsrichtung einen Winkel > 0°, vorzugweise zwischen 60° und 120° einschließt, und/oder
c. die Betätigungseinheit (30) weist ein Applikatorrohr (34) auf, an dessen distalem Ende die Austragöffnung (38) vorgesehen ist, wobei der Spender (10) vorzugweise für den sublingualen Austrag von Flüssigkeit vorgesehen ist, und hierfür insbesondere vorzugsweise eine angewinkelte Applikatorrohrspitze (34B) aufweist.

12. Pumpspender (10) nach Anspruch 6 oder 11 oder Set nach Anspruch 10 oder 11 mit mindestens einem der folgenden weiteren Merkmale:
a. der Flüssigkeitsspeicher (46) ist mit einer pharmazeutischen Flüssigkeit befüllt, und/oder
b. der Flüssigkeitsspeicher (46) weist ein Volumen zwischen 20 ml und 1000 ml auf, vorzugweise zwischen 50 ml und 500 ml, und/oder
c. die Pumpe (20) ist als Kolbenpumpe mit einem Pumpenzylinder und einem darin verschieblichen Pumpenkolben ausgebildet, die gemeinsam die Pumpenkammer begrenzen, wobei vorzugsweise eingangsseitig und ausgangseitig der Pumpenkammer druckabhängig öffnende und schließende Ventile vorgesehen sind.

13. Pumpspender (10) nach einem der Ansprüche 6 oder 11 bis 12 oder Auswertungseinheit (50, 70) nach einem der Ansprüche 7 bis 9 mit mindestens einem der folgenden weiteren Merkmale:
a. der Kraftsensor (64, 84) ist als FSR-Sensor (Force Sensitive Resistor) oder als Piezo-Sensor ausgebildet, und/oder
b. dem Kraftsensor (64, 84) ist eine Feder (58, 78) zugeordnet, die insbesondere vorzugsweise in einem entspannten Zustand die auf den Kraftsensor (64, 84) wirkenden Bauteile derart voneinander beabstandet, dass eines der Bauteile den Kontakt zum Kraftsensor (64, 84) verliert, und/oder
c. die Auswertungseinheit (50, 70) umfasst mindestens einen weiteren Sensor (87), insbesondere einen Beschleunigungs- und/oder Lagesensor, und/oder
d. die Auswertungseinheit (50, 70) umfasst eine Energiequelle (66, 86) in Form einer Batterie, insbesondere einer wiederaufladbaren Batterie, und/oder
e. die Auswertungseinheit (50, 70) umfasst eine Funkschnittstelle (63, 83), insbesondere eine WLAN-Schnittstelle, eine NFC-Schnittstelle oder eine Bluetooth-Schnittstelle.

## Claims

1. Method for evaluating a pump actuation on a pump dispenser (10) for discharging a fluid, in particular a pharmaceutical fluid, having the following features:
a. the method is applied to a pump dispenser (10) which has two sub-units (12, 14), which can be moved manually relative to one another, and has a pump (20) having a pump chamber that can be made smaller by moving the sub-units (12, 14) from an unactuated first end position into an actuated second end position, and
b. to detect the actuated second end position, a force with which the sub-units (12, 14) are manually moved towards one another is detected by means of a force sensor (64, 84) during the movement of the sub-units (12, 14) towards one another, and
c. the time curve of the detected force is evaluated in such a way that a force increase characteristic of reaching the actuated second end position is identified, and
d. depending on whether the force increase characteristic of reaching the actuated second end position has been detected, a signal characteristic of the result is output haptically, acoustically and/or visually and/or is sent via a wireless interface to an external display device by an output device,
**characterized by** the following further features:
e. the data detected by the force sensor (64, 84) are stored in a memory in such a way that the time curve of the detected force results from this, and
f. the evaluation used to determine whether a force increase characteristic of reaching the actuated second end position has occurred is performed taking the detected time curve into account.

2. Method according to Claim 1, **characterized by** the following further features:
a. the evaluation is performed using a processor (62, 82) which has an operating mode as well as a power-saving sleep mode, and
b. the force sensor (64, 84) is connected to the processor in such a way that the processor (62, 82) in the sleep mode switches into the operating mode once a predetermined limit value is exceeded, and
c. subsequently, in the operating mode, the values detected by the force sensor (64, 84) are stored in the memory and repeatedly analyzed by the processor (62, 82) to identify the force increase characteristic of reaching the actuated second end position.

3. Method according to Claim 1 or 2, **characterized by** at least one of the following further features:
a. the evaluation of the force over time is performed using a smoothed graph of the force over time,
and/or
b. the evaluation of the force over time is performed in such a way that the slope of the force over time is evaluated, wherein an increase in the slope is interpreted as an indicator for reaching the second end position, and wherein in particular a quotient of the slope and the force value is compared with a limit value and exceeding this limit value is interpreted as reaching the second end position, wherein in particular the limit value is time-dependent or force-dependent and decreases over time,
and/or
c. the evaluation of the force over time is performed in such a way that the force detected by the force sensor is used to categorize the actuation into one of at least two categories that are assigned to actuations of different strengths and/or speeds, wherein the reaching of the second actuated end position and/or the time of reaching the second actuated end position are identified in different ways for the at least two categories.

4. Method according to one of the preceding claims, **characterized by** at least one of the following further features:
a. the signaling is performed in such a way that after the start of the actuation a first signal is output, which ends as soon as the force increase characteristic of reaching the actuated second end position is detected and/or as soon as a force curve or force value characteristic of an end of the actuation has been measured, and
b. the signaling is performed in such a way that a second signal is output when the force increase characteristic of reaching the actuated second end position has been detected, and
c. the signaling is performed in such a way that a third signal is output as soon as a force curve or force value characteristic of an end of actuation has been measured.

5. Method according to one of the preceding claims, **characterized by** the following further feature:
a. the method provides that the measurement data and/or data for detecting the force increase, detected by means of the force sensor (64, 84), are sent to the external display device (100) via the wireless interface (63, 83),
in particular with at least one of the following additional features:
b. the external display device (100) indicates to the user whether the second actuated end position was achieved with the last discharge, and/or
c. the display device sends the detected measurement data and/or data for detecting or not detecting the force increase characteristic of reaching the second actuated end position to an external server (102), in particular for transmission to a physician.

6. Pump dispenser (10) for discharging a fluid, in particular for discharging a pharmaceutical fluid, having the following features
a. the pump dispenser (10) has a fluid reservoir (46) for receiving the fluid prior to discharge and at least one discharge opening (38) through which fluid can be discharged into an environment, and
b. the pump dispenser (10) has an actuating pusher (32) which is displaceable relative to the fluid reservoir (46),
c. the pump dispenser (10) has a pump (20) having a pump chamber, wherein walls of the pump chamber are movable relative to one another between an unactuated first end position and an actuated second end position for the purpose of reducing the size of the pump chamber, and wherein a first wall of the pump chamber is provided stationary relative to the fluid reservoir (46) and a second wall of the pump chamber is displaceable relative to the first wall by manual actuation of the actuating pusher (32), and
d. the pump dispenser (10) has an evaluation unit (50, 70) for identifying when the second actuated end position has been reached by means of the method according to one of Claims 1 to 5, wherein
- the evaluation unit (50, 70) has a force sensor (64, 84), which measures the force applied by a user between the actuating pusher (32) and the second wall or between a retaining surface (48) on the fluid reservoir (46) and the first wall, and
- the evaluation unit (50, 70) has a processor (62, 82), which evaluates the force detected by the force sensor (64, 84) so that a force increase characteristic of reaching the actuated second end position is detected, and
- the evaluation unit (50, 70) has an output device (68, 88), which, depending on the force evaluation, outputs a signal characteristic of the result of the force evaluation as a haptic, acoustic and/or visual signal, and/or the evaluation unit (50, 70) has a wireless interface (63, 83), which, as a function of the force evaluation, can send a signal characteristic of the result of the force evaluation to an external display device (100).

7. Evaluation unit (50, 70) for a pump dispenser for discharging a fluid, in particular for discharging a pharmaceutical fluid, having the following features:
a. the evaluation unit (50, 70) is provided for tool-free attachment to a pump dispenser (10), which has a fluid reservoir (46) for receiving the fluid prior to discharge, at least one discharge opening (38) through which fluid can be discharged into an environment, and an actuating pusher (32) which is displaceable relative to the fluid reservoir (46), wherein the pump dispenser (10) further has a pump (20) which is actuatable by displacement of the actuating pusher (32) relative to the fluid reservoir (46) between an unactuated first end position and an actuated second end position, and
b. the evaluation unit (50, 70) is designed to identify when the second actuated end position has been reached by means of the method according to one of Claims 1 to 5, wherein
- the evaluation unit (50) is designed to be attached to the fluid reservoir (46) and has a fastening region (56) for stationary contact with the fluid reservoir (46) and a retaining surface (53) for manual gripping of the evaluation unit (50), wherein the evaluation unit further has a force sensor (64) for detecting the force applied by a user between the retaining surface (53) and the fastening region (56),
or
- the evaluation unit (70) is designed to be attached to the actuating pusher (32) and has a fastening region (76) for stationary contact with the actuating pusher (32) and an actuating surface (73) for manually pressing down the evaluation unit (70), wherein the evaluation unit (70) further has a force sensor (84) which detects the force applied by a user between the actuating surface (73) and the fastening region (76),
and
- the evaluation unit (50, 70) has a processor (62, 82), which evaluates the force detected by the force sensor (64, 84) so that a force increase characteristic of reaching the actuated second end position is identified, and
- the evaluation unit (50, 70) has an output device (68, 88), which, as a function of the force evaluation, outputs a signal characteristic of the result of the force evaluation as a haptic, acoustic and/or visual signal and/or the evaluation unit has a wireless interface (63, 83), which, as a function of the force evaluation, can send a signal characteristic of the result of the force evaluation to an external display device (100).

8. Evaluation unit (50) according to Claim 7 with the following further features:
a. the evaluation unit (50) is designed to be attached to the fluid reservoir (46), and
b. the evaluation unit has a housing (51) with a casing wall (52) into which the fluid reservoir (46) of the pump dispenser (10) can be inserted and the outside of which forms the retaining surface (53), and
c. the fastening region (56) of the evaluation unit is provided inside the casing wall (52) and comes into contact there with the inserted pump dispenser (10).

9. Evaluation unit (50) according to Claim 8 with the following further feature:
d. the fastening region (56) is provided with at least one elastically deflectable retaining element (56A) and is designed to receive the pump dispenser (10) in such a way that, when the pump dispenser (10) is inserted into the fastening region (56), the retaining element (56A) is elastically deflected and thereby produces a retaining force.

10. Set consisting of a pump dispenser and an evaluation unit with the following features:
a. the set has a pump dispenser (10) with a fluid reservoir (46) and an actuating pusher (32) movable relative to the fluid reservoir (46), and
b. the set has an evaluation unit (50, 70) according to one of Claims 7 to 9 to be attached to the fluid reservoir (46) or to the actuating pusher (32) of the pump dispenser (10).

11. Pump dispenser (10) according to Claim 6 or set according to Claim 10, **characterized by** the additional feature:
a. the pump dispenser (10) has an actuating unit (30) which comprises the actuating pusher (32) and the discharge opening (38) and which as a whole is displaceable in an actuation direction relative to the fluid reservoir (46) for the purpose of actuating the pump (20),
in particular with at least one of the additional features:
b. the discharge opening (38) is provided laterally on the actuating unit (30), so that a discharge direction forms an angle > 0° with the actuating direction, preferably between 60° and 120°, and/or
c. the actuating unit (30) has an applicator tube (34), at the distal end of which the discharge opening (38) is provided, wherein the dispenser (10) is preferably provided for the sublingual discharge of fluid, and for this purpose particularly preferably has an angled applicator tube tip (34B).

12. Pump dispenser (10) according to Claim 6 or 11 or set according to Claim 10 or 11 with at least one of the following further features:
a. the fluid reservoir (46) is filled with a pharmaceutical fluid, and/or
b. the fluid reservoir (46) has a volume between 20 ml and 1000 ml, preferably between 50 ml and 500 ml, and/or
c. the pump (20) is designed as a piston pump with a pump cylinder and a pump piston displaceable therein, which together delimit the pump chamber, wherein pressure-dependent opening and closing valves are preferably provided on the inlet side and outlet side of the pump chamber.

13. Pump dispenser (10) according to one of Claims 6 or 11 to 12 or evaluation unit (50, 70) according to one of Claims 7 to 9 with at least one of the following further features:
a. the force sensor (64, 84) is designed as an FSR sensor (Force Sensitive Resistor) or as a piezo sensor, and/or
b. a spring (58, 78) is associated with the force sensor (64, 84) and, particularly preferably in a relaxed state, spaces the components acting on the force sensor (64, 84) from one another in such a way that one of the components loses contact with the force sensor (64, 84), and/or
c. the evaluation unit (50, 70) comprises at least one further sensor (87), in particular an acceleration and/or position sensor, and/or
d. the evaluation unit (50, 70) comprises an energy source (66, 86) in the form of a battery, in particular a rechargeable battery, and/or
e. the evaluation unit (50, 70) comprises a radio interface (63, 83), in particular a WLAN interface, an NFC interface or a Bluetooth interface.

## Revendications

1. Procédé d'évaluation du fonctionnement d'une pompe sur un distributeur à pompe (10) destiné à délivrer un liquide, notamment un liquide pharmaceutique, ledit procédé comprenant les caractéristiques suivantes :
a. le procédé est utilisé sur un distributeur à pompe (10) qui dispose de deux sous-unités (12, 14) pouvant être déplacées manuellement l'une par rapport à l'autre et qui dispose d'une pompe (20) comprenant une chambre de pompe qui peut être réduite par le mouvement des sous-unités (12, 14) d'une première position finale 'non-actionnées' jusque dans une deuxième position finale 'actionnées', et
b. pour détecter la deuxième position finale 'actionnées', une force avec laquelle les sous-unités (12, 14) sont déplacées manuellement l'un vers l'autre est détectée lors du mouvement des sous-unités (12, 14) l'une par rapport à l'autre au moyen d'un capteur de force (64, 84), et
c. la variation dans le temps de la force détectée est évaluée par détection d'une augmentation de la force caractéristique pour atteindre la deuxième position finale dans les sous-unités sont actionnées, et
d. en fonction de la détection de l'augmentation de la force caractéristique pour atteindre la deuxième position finale dans laquelle les sous-unités sont actionnées, un signal caractéristique du résultat est émis de manière haptique, acoustique et/ou visuelle par un dispositif de sortie et/ou est envoyé à un appareil de présentation extérieur par le biais d'une interface sans fil,
**caractérisé par** les caractéristiques supplémentaires suivantes :
e. les données acquises par le capteur de force (64, 84) sont stockées dans une mémoire de manière à obtenir la variation dans le temps de la force détectée, et
f. l'évaluation, avec laquelle il est déterminé si une augmentation de force caractéristique pour atteindre la deuxième position finale dans laquelle les sous-unités sont actionnées se produit, est effectuée avec prise en compte la variation dans le temps détectée.

2. Procédé selon la revendication 1, **caractérisé par** les caractéristiques supplémentaires suivantes :
a. l'évaluation est effectuée à l'aide d'un processeur (62, 82) qui dispose d'un mode de fonctionnement et d'un mode de repos qui est par contre à économie d'énergie, et
b. le capteur de force (64, 84) est relié au processeur de manière à ce que le processeur (62, 82) en mode de fonctionnement passe en mode de repos après franchissement vers le haut d'une valeur limite spécifiée, et
c. ensuite, en mode de fonctionnement, les valeurs acquises par le capteur de force (64, 84) sont stockées dans la mémoire et analysées à plusieurs reprises par le processeur (62, 82) afin de détecter l'augmentation de force caractéristique pour atteindre la deuxième position finale 'actionnées'.

3. Procédé selon la revendication 1 ou 2, **caractérisé par** l'une au moins des caractéristiques supplémentaires suivantes :
a. l'évaluation de la force dans le temps est effectuée par référence d'un graphique lissé de la force dans le temps,
et/ou
b. l' évaluation de la force dans le temps est effectuée de manière à évaluer la pente de la force dans le temps, la montée de la pente étant interprétée comme un indicateur pour atteindre la deuxième position finale et en particulier un quotient formé par la pente et la valeur de force étant comparé à une valeur limite et le franchissement vers le haut de cette valeur limite étant interprété comme l'atteinte de la deuxième position finale, en particulier la valeur limite dépendant du temps ou de la force et diminuant dans le temps,
et/ou
c. l'évaluation de la force dans le temps est effectuée de manière à réaliser une catégorisation de l'actionnement dans l'une des deux catégories, qui sont associées à des actionnements différents en intensité et/ou en vitesse, sur la base de la force détectée par le capteur de force, la détection du fait que la deuxième position finale dans laquelle les sous-unités sont actionnées est atteinte et/ou la détection de l'instant où la deuxième position finale dans laquelle les sous-unités sont actionnées sont effectuées de différentes manières pour les au moins deux catégories.

4. Procédé selon l'une des revendications précédentes, **caractérisé par** l'une au moins des caractéristiques supplémentaires suivantes :
a. la signalisation est effectuée de manière à ce que, après le début de l'actionnement, un premier signal soit émis qui se termine dès que l'd'augmentation de force caractéristique pour atteindre la deuxième position finale 'actionnées' est détectée et/ou dès qu'une variation de force ou valeur de force caractéristique d'une fin de l'actionnement a été mesurée, et
b. la signalisation est effectuée de manière à ce qu'un deuxième signal soit émis lorsque l'augmentation de force caractéristique pour atteindre la deuxième position finale 'actionnées' a été détectée, et
c. la signalisation est effectuée de manière à ce qu'un troisième signal soit émis dès qu'une variation de force ou valeur de force caractéristique de la fin de l'actionnement a été mesurée.

5. Procédé selon l'une des revendications précédentes, **caractérisé par** la caractéristique supplémentaire suivante :
a. le procédé prévoit d'envoyer les données de mesure acquises par le capteur de force (64, 84) et/ou les données relatives à la détection de l'augmentation de force à l'appareil de présentation extérieur (100) par le biais de l'interface sans fil (63, 83),
notamment avec au moins une des caractéristiques supplémentaires suivantes :
b. l'appareil de présentation extérieur (100) indique à l'utilisateur si la deuxième position finale dans laquelle les sous-unités sont actionnées a été atteinte lors de la dernière délivrance et/ou
c. l'appareil de présentation envoie à un serveur extérieur (102), en particulier pour la transmission à un médecin, les données de mesure acquises et/ou données acquises de détection ou non-détection de l'augmentation de force caractéristique pour atteindre la deuxième position finale 'actionnées'.

6. Distributeur à pompe (10) destiné à la délivrance d'un liquide, notamment la délivrance d'un liquide pharmaceutique, ledit distributeur à pompe présentant les caractéristiques suivantes :
a. le distributeur à pompe (10) dispose d'un réservoir de liquide (46) destiné à recevoir le liquide avant la délivrance et d'au moins une ouverture de délivrance (38) par laquelle le liquide peut être délivré dans un environnement, et
b. le distributeur à pompe (10) dispose d'un poussoir d'actionnement (32) qui peut être déplacé par rapport au réservoir de liquide (46),
c. le distributeur à pompe (10) dispose d'une pompe (20) comprenant une chambre de pompe, des parois de la chambre de pompe étant mobiles les unes par rapport aux autres entre une première position finale 'non-actionnées' et une deuxième position finale 'actionnées' afin de réduire la chambre de pompe et une première paroi de la chambre de pompe est prévue de manière fixe par rapport au réservoir de liquide (46) et une deuxième paroi de la chambre de pompe peut être déplacée par rapport à la première paroi par actionnement manuel du poussoir d'actionnement (32), et
d. le distributeur à pompe (10) comporte une unité d'évaluation (50, 70) destinée à détecter si la deuxième position finale 'actionnées' a été atteinte à l'aide du procédé selon l'une des revendications 1 à 5,
- l'unité d'évaluation (50, 70) comportant un capteur de force (64, 84) qui mesure la force, appliquée par un utilisateur, entre le poussoir d'actionnement (32) et la deuxième paroi ou entre une surface de retenue (48) sur le réservoir de liquide (46) et la première paroi, et
- l'unité d'évaluation (50, 70) comportant un processeur (62, 82) qui évalue la force acquise par le capteur de force (64, 84) de manière à détecter une augmentation de force caractéristique pour atteindre la deuxième position finale 'actionnées', et
- l'unité d'évaluation (50, 70) disposant d'un dispositif de sortie (68, 88) qui, en fonction de l'évaluation de la force, délivre un signal caractéristique du résultat de l'évaluation de la force sous la forme d'un signal haptique, acoustique et/ou visuel et/ou l'unité d'évaluation (50, 70) disposant d'une interface sans fil (63, 83) qui, en fonction de l'évaluation de la force, peut envoyer un signal caractéristique du résultat de l'évaluation de la force à un appareil de présentation extérieur (100).

7. Unité d'évaluation (50, 70) destinée à un distributeur à pompe destiné à délivrer un liquide, notamment délivrer un liquide pharmaceutique, ladite unité d'évaluation présentant les caractéristiques suivantes :
a. l'unité d'évaluation (50, 70) est prévue pour être montée sans outil sur un distributeur à pompe (10) qui dispose d'un réservoir de liquide (46) destiné à recevoir le liquide avant la délivrance, d'au moins une ouverture de délivrance (38) par laquelle le liquide pouvant être délivré dans un environnement et d'un poussoir d'actionnement (32) qui peut être déplacé par rapport au réservoir de liquide (46), le distributeur à pompe (10) disposant en outre d'une pompe (20) qui peut être actionnée par déplacement du poussoir d'actionnement (32) par rapport au réservoir de liquide (46) entre une première position finale 'non-actionnée' et une deuxième position finale 'actionnée', et
b. l'unité d'évaluation (50, 70) est conçue pour détecter que la deuxième position finale 'actionnée' est atteinte au moyen du procédé selon l'une des revendications 1 à 5,
- l'unité d'évaluation (50) est conçue pour être montée sur le réservoir de liquide (46) et dispose d'une zone de fixation (56) destinée à venir en appui fixe sur le réservoir de liquide (46) ainsi que d'une surface de retenue (53) destinée à saisir manuellement l'unité d'évaluation (50), l'unité d'évaluation disposant en outre d'un capteur de force (64) qui détecte la force, appliquée par un utilisateur, entre la surface de retenue (53) et la zone de fixation (56),
ou
l'unité d'évaluation (70) est conçue pour être montée sur le poussoir d'actionnement (32) et dispose d'une zone de fixation (76) destinée à venir en appui fixe sur le poussoir d'actionnement (32) ainsi que d'une surface d'actionnement (73) destiné à affaisser manuellement l'unité d'évaluation (70), l'unité d'évaluation (70) disposant en outre d'un capteur de force (84) qui détecte la force, appliquée par un utilisateur, entre la surface d'actionnement (73) et la zone de fixation (76),
et
- l'unité d'évaluation (50, 70) dispose d'un processeur (62, 82) qui évalue la force détectée par le capteur de force (64, 84) de manière à détecter une augmentation de force caractéristique pour atteindre la deuxième position finale 'actionnée', et
- l'unité d'évaluation (50, 70) dispose d'un dispositif de sortie (68, 88) qui, en fonction de l'évaluation de la force, délivre un signal caractéristique du résultat de l'évaluation de la force sous la forme d'un signal haptique, acoustique et/ou visuel et/ou l'unité d'évaluation dispose d'une interface sans fil (63, 83) qui, en fonction de l'évaluation de la force, peut envoyer un signal caractéristique du résultat de l'évaluation de la force à un appareil de présentation extérieur (100).

8. Unité d'évaluation (50) selon la revendication 7 comprenant les caractéristiques supplémentaires suivantes :
a. l'unité d'évaluation (50) est conçue pour être montée sur le réservoir de liquide (46), et
b. l'unité d'évaluation comporte un boîtier (51) pourvue d'une paroi d'enveloppe (52) dans lequel le réservoir de liquide (46) du distributeur à pompe (10) peut être inséré et dont l'extérieur forme la surface de retenue (53), et
c. la zone de fixation (56) de l'unité d'évaluation est prévue à l'intérieur de la paroi d'enveloppe (52) et vient en contact avec le distributeur à pompe (10) inséré.

9. Unité d'évaluation (50) selon la revendication 8 comprenant la caractéristique supplémentaire suivante :
d. la zone de fixation (56) est pourvue d'au moins un élément de retenue (56A) qui peut être dévié élastiquement et est conçue pour recevoir le distributeur à pompe (10) de manière à ce que, lorsque le distributeur à pompe (10) est inséré dans la zone de fixation (56), l'élément de retenue (56A) soit dévié élastiquement et génère ainsi une force de retenue.

10. Ensemble formé d'un distributeur à pompe et d'une unité d'évaluation, ledit ensemble présentant les caractéristiques suivantes :
a. l'ensemble dispose d'un distributeur à pompe (10) comprenant un réservoir de liquide (46) et un poussoir d'actionnement (32) mobile par rapport au réservoir de liquide (46), et
b. l'ensemble dispose d'une unité d'évaluation (50, 70) selon l'une des revendications 7 à 9 destinée à être montée sur le réservoir de liquide (46) ou la poussoir d'actionnement (32) du distributeur à pompe (10).

11. Distributeur à pompe (10) selon la revendication 6 ou ensemble selon la revendication 10, **caractérisé par** la caractéristique supplémentaire suivante :
a. le distributeur à pompe (10) dispose d'une unité d'actionnement (30) qui comprend le poussoir d'actionnement (32) et l'ouverture de délivrance (38) et qui peut être déplacée dans son ensemble dans une direction d'actionnement par rapport au réservoir de liquide (46) afin d'actionner la pompe (20), notamment avec au moins une des caractéristiques supplémentaires suivantes :
b. l'ouverture de délivrance (38) est prévue sur le côté de l'unité d'actionnement (30) de sorte qu'une direction de délivrance forme un angle > 0°, de préférence entre 60° et 120°, avec la direction d'actionnement, et/ou
c. l'unité d'actionnement (30) comporte un tube applicateur (34) à l'extrémité distale duquel est prévue l'ouverture de délivrance (38), le distributeur (10) étant de préférence prévu pour la délivrance sublinguale de liquide et comportant pour cela, de préférence, une pointe de tube applicateur coudée (34B).

12. Distributeur à pompe (10) selon la revendication 6 ou 11 ou ensemble selon la revendication 10 ou 11 comprenant l'une au moins des caractéristiques supplémentaires suivantes :
a. le réservoir de liquide (46) est rempli d'un liquide pharmaceutique et/ou
b. le réservoir de liquide (46) a un volume compris entre 20 ml et 1000 ml, de préférence entre 50 ml et 500 ml, et/ou
c. la pompe (20) est conçue comme une pompe à piston comprenant un cylindre de pompe et un piston de pompe mobile à l'intérieur qui délimitent conjointement la chambre de pompe, des clapets d'ouverture et de fermeture dépendant de la pression étant de préférence prévues du côté entrée et du côté sortie de la chambre de pompe.

13. Distributeur à pompe (10) selon l'une des revendications 6 ou 11 à 12 ou unité d'évaluation (50, 70) selon l'une des revendications 7 à 9 comprenant l'une au moins des caractéristiques supplémentaires suivantes :
a. le capteur de force (64, 84) est conçu comme un capteur FSR (Force Sensitive Resistor) ou comme un capteur piézo, et/ou
b. un ressort (58, 78) est associé au capteur de force (64, 84), lequel capteur éloigne, notamment de préférence à l'état détendu, les composants qui agissent sur le capteur de force (64, 84) les uns des autres de manière à ce que l'un des composants perde la contact avec le capteur de force (64, 84), et/ou
c. l'unité d'évaluation (50, 70) comprend au moins un autre capteur (87), en particulier un capteur d'accélération et/ou de position, et/ou
d. l'unité d'évaluation (50, 70) comprend une source d'énergie (66, 86) se présentant sous la forme d'une batterie, en particulier une batterie rechargeable, et/ou
e. l'unité d'évaluation (50, 70) comprend une interface radio (63, 83), en particulier une interface WLAN, une interface NFC ou une interface Bluetooth.
